(19) Europäisches Patentamt
European Patent Office
Office européen des brevets

(11) Publication number: 0 214 462
A2

(12) EUROPEAN PATENT APPLICATION

(21) Application number: 86110728.2

(22) Date of filing: 02.08.86

(51) Int. Cl.[4]: C 07 D 501/36
A 61 K 31/545

A request pursuant to Rule 88 EPC for correction of the claims has been filed on 11.10.86. A decision will be taken by the Examing Division (Guidelines for Examination in the EPO, A V 2.2).

(30) Priority: 05.08.85 GB 8519606
17.03.86 GB 8606544
02.06.86 GB 8613268

(43) Date of publication of application: 18.03.87 Bulletin 87/12

(84) Designated Contracting States: AT BE CH DE FR GB IT LI LU NL SE

(71) Applicant: Fujisawa Pharmaceutical Co., Ltd.
3, Doshomachi 4-chome Higashi-ku
Osaka-shi, Osaka 541(JP)

(72) Inventor: Takaya, Takao
No. 1-5-87, Suimeidai
Kawanishi(JP)

(72) Inventor: Inamoto. Yoshiko
No. 4-7-16-810, Uenonishi
Toyonaka(JP)

(74) Representative: Türk, Dietmar, Dr. rer. nat. et al,
Türk, Gille + Hrabal Patentanwälte Bruckner Strasse 20
D-4000 Düsseldorf 13(DE)

(54) 3,7-Disubstituted-3-cephem compounds and processes for production of the same.

(57) A cephem compound of the formula:

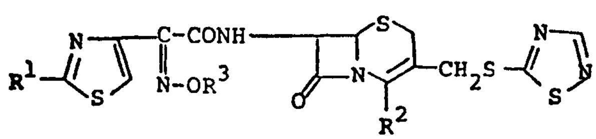

wherein $R^1$ is amino or protected amino,
$R^2$ is carboxy or protected carboxy, and
$R^3$ is hydrogen or hydroxy-protective group,
and pharmaceutically acceptable salts thereof processes for their preparation and pharmaceutical compositions comprising them. The invention relates also to compounds of the formula

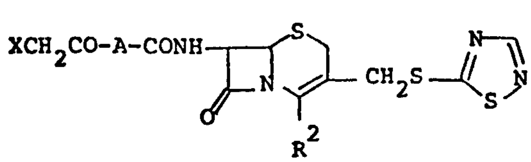

and processes for their preparation.



Croydon Printing Company Ltd.

# 3,7-DISUBSTITUTED-3-CEPHEM COMPOUNDS AND PROCESSES FOR PRODUCTION OF THE SAME

The present invention relates to novel 3,7-disubstituted-3-cephem compounds and a pharmaceutically acceptable salt thereof.

More particularly, it relates to novel 3,7-disubstituted-3-cephem compounds and a pharmaceutically acceptable salt thereof, which have antimicrobial activity, to processes for the production of the same, to a pharmaceutical composition comprising the same, and to a method for the treatment of infectious diseases caused by pathogenic microorganisms comprising administering the same to infected human being or animals.

Accordingly, one object of the present invention is to provide novel 3,7-disubstituted-3-cephem compounds and a pharmaceutically acceptable salt thereof, which are highly active against a number of pathogenic microorganisms and are useful as antimicrobial agents.

Another object of the present invention is to provide processes for the production of novel 3,7-disubstituted-3-cephem compounds and a salt thereof.

A further object of the present invention is to provide a pharmaceutical composition comprising, as an active ingredient, said 3,7-disubstituted-3-cephem compounds and a pharmaceutically acceptable salt thereof.

Still further object of the present invention is to provide a method for the treatment of infectious diseases caused by pathogenic microorganisms which comprises administering said 3,7-disubstituted-3-cephem compounds and a pharmaceutically acceptable salt thereof to the infected human being or animals.

With regard to the states of the arts of the present invention, for example, the following compounds are known.

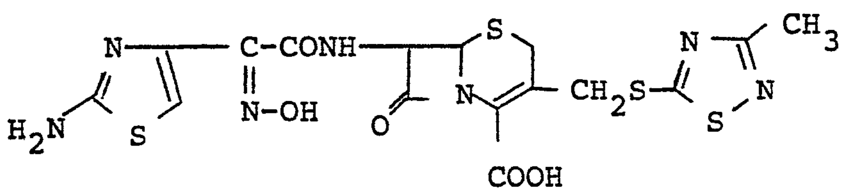

(A)

(U.S. Patent No. 4,264,595)

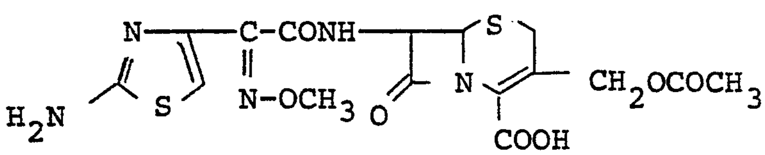

(B)

(Cefotaxime)

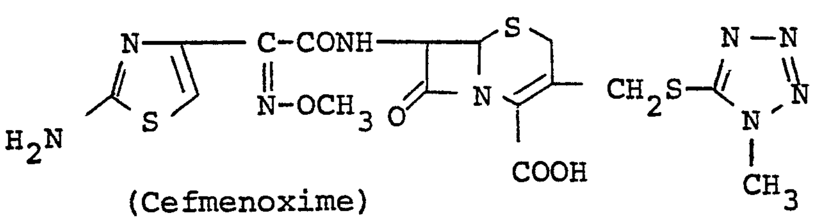

(C)

(Cefmenoxime)

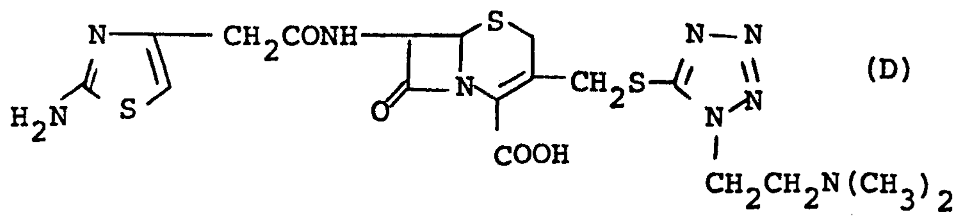

(D)

(Cefotiam)

However, antimicrobial spectra of such known compounds are restrictive and especially the antimicrobial activities thereof against Gram positive bacteria are not so potent.

Under such a situation, antimicrobial agents having broad antimicrobial spectra, especially having potent antimicrobial activities against Gram positive bacteria as well are strongly wanted.

And, as a result of an extensive study, the inventors of the present invention have succeeded in obtaining such superior antimicrobial agents.

The novel 3,7-disubstituted-3-cephem compounds according to this invention can be represented by the following general formula (I).

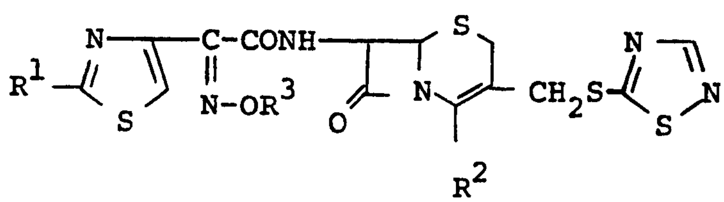

wherein $R^1$ is amino or protected amino,

$R^2$ is carboxy or protected carboxy, and

$R^3$ is hydrogen or hydroxy-protective group.

0214402

It is to be understood that the stereospecific partial structure of the formula :

$$\begin{array}{l} -C-CO- \\ \;\| \\ \;N-OR^3 \end{array}$$

, wherein $R^3$ is as defined above,

of the compound (I) is entitled a syn form in the present specification, and the term "syn isomer" means the compound having the above stereospecific structure.

Suitable salts of the object compound (I) are conventional non-toxic, pharmaceutically acceptable salts and may include a salt with a base or an acid addition salt such as a salt with an inorganic base, for example, an alkali metal salt (e.g. sodium salt, potassium salt, cesium salt, etc.), an alkaline earth metal salt (e.g. calcium salt, magnesium salt, etc.), an ammonium salt; a salt with an organic base, for example, an organic amine salt (e.g. triethylamine salt, pyridine salt, picoline salt, ethanolamine salt, triethanolamine salt, dicyclohexylamine salt, N,N'-dibenzylethylenediamine salt, etc.), etc.; an inorganic acid addition salt (e.g. hydrochloride, hydrobromide, sulfate, phosphate, etc.); an organic carboxylic or sulfonic acid addition salt (e.g. formate, acetate, trifluoroacetate, maleate, tartrate, methanesulfonate, benzenesulfonate, p-toluenesulfonate, etc.); a salt with a basic or acidic amino acid (e.g. arginine, aspartic acid, glutamic acid, etc.); and the like.

The object compound (I) or a salt thereof of the invention can be prepared by the processes illustrated in the following reaction schemes.

Process 1 :

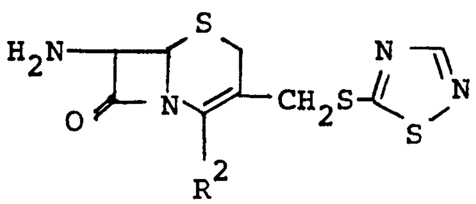

(II)

or its reactive derivative at the amino group or a salt thereof

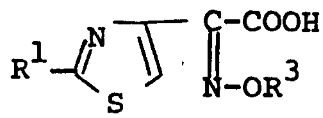

(III)

or its reactive derivative at the carboxy group or a salt thereof

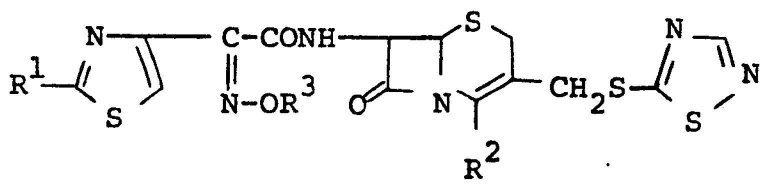

(I)

or a salt thereof

Process 2 :

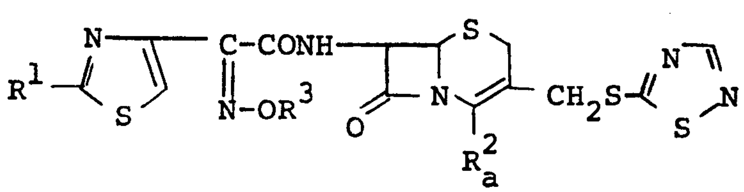

(Ia)

or a salt thereof

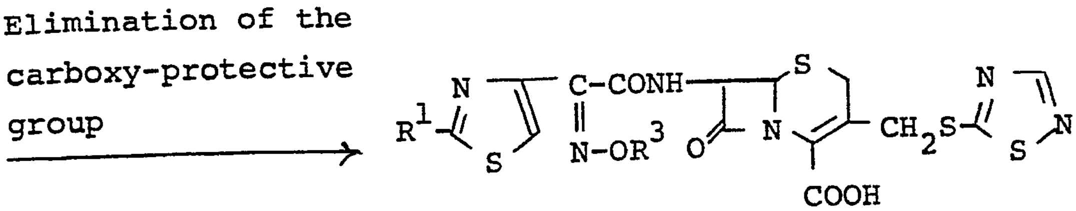


(Ib)

or a salt thereof

Process 3 :

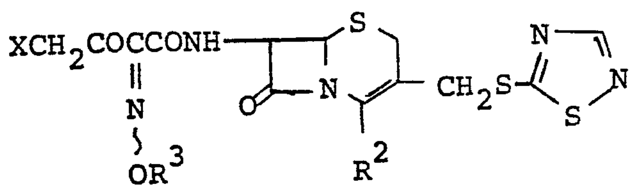

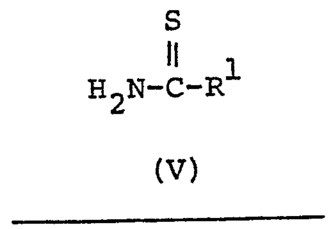

(V)

(IV)

or a salt thereof

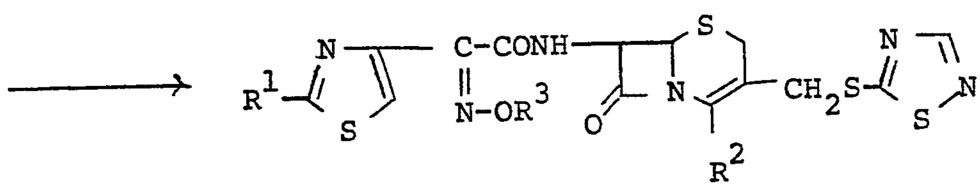

(I)

or a salt thereof

Process 4 :

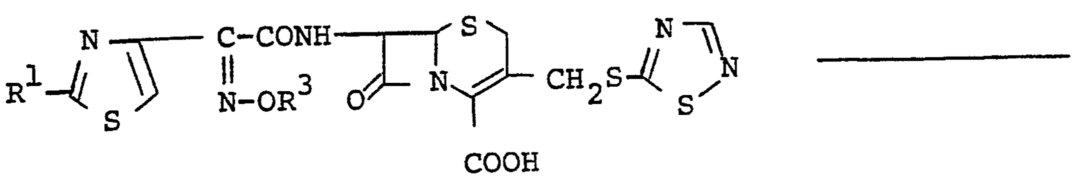

(Ib)

or a salt thereof

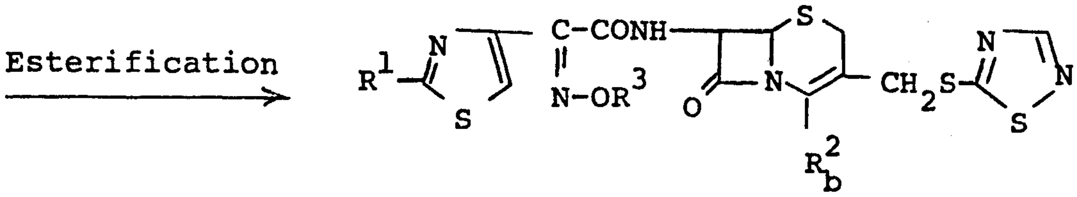


(Ic)

or a salt thereof

Process 5 :

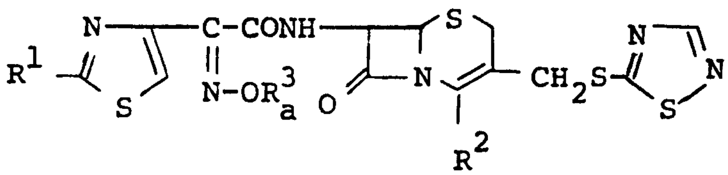

(Id)

or a salt thereof

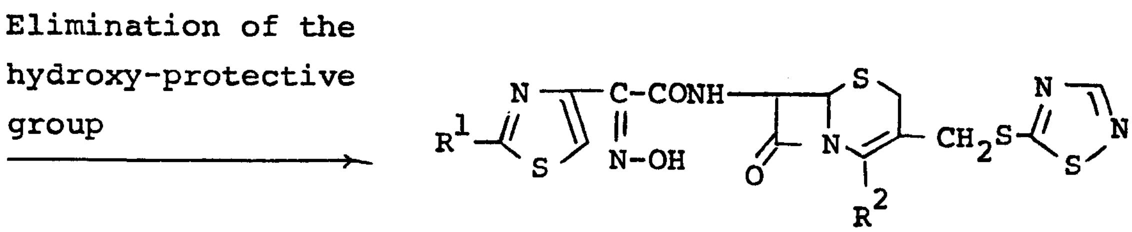


(Ie)

or a salt thereof

Process 6 :

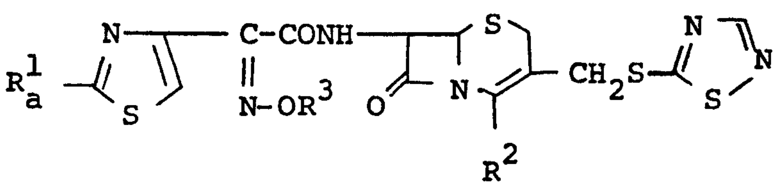

(If)

or a salt thereof

Elimination of the amino-protective group

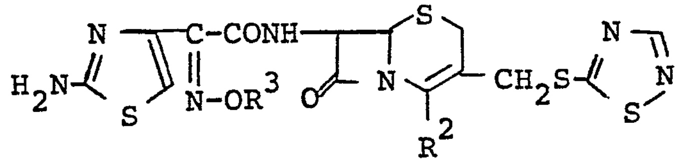

(Ig)

or a salt thereof

Process 7 :

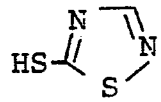

(IX)

or its reactive derivative at the mercapto group

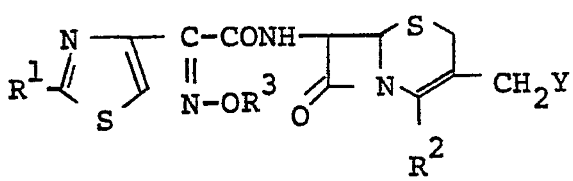

(VI)

or a salt thereof

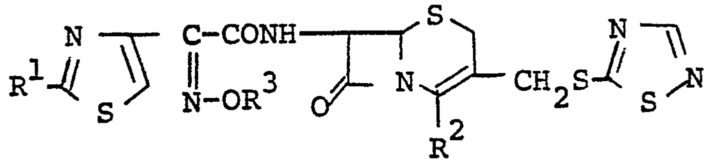

(I)

or a salt thereof

wherein $R^1$, $R^2$ and $R^3$ are each as defined above,

$R^1_a$ is protected amino,

$R^2_a$ is protected carboxy,

$R^2_b$ is esterified carboxy,

$R^3_a$ is hydroxy-protective group,

X is an acid residue, and

Y is a group which is replaceable with the formula :

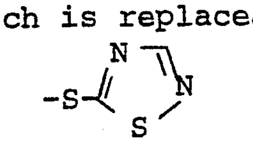

Among the starting compounds in the present invention, the compound (IV) and its intermediate are novel and can be represented by the following general formula :

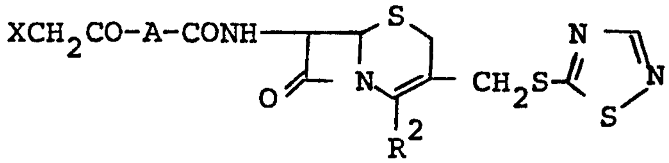

(IV')

wherein $R^2$ and X are each as defined above, and

A is methylene or a group of the formula : $=N\sim OR^3$, in which $R^3$ is hydrogen or hydroxy-protective group,

or a salt thereof, and can be prepared by the processes illustrated in the following schemes or by a conventional method.

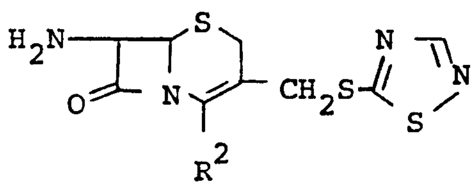

(II)

or a reactive derivative at the amino group thereof, or a salt thereof

Process (1)

$X-CH_2CO-A-COOH$ (VII)

or a reactive derivative at the carboxy group or a salt thereof

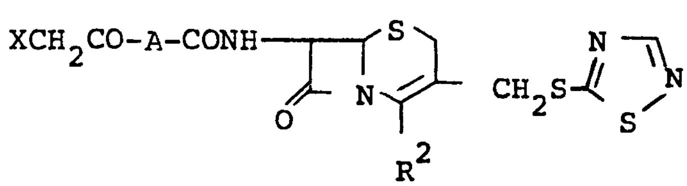

(VIII)

or a salt thereof

Process (2)

Nitrosating Agent
(wherein A is methylene)

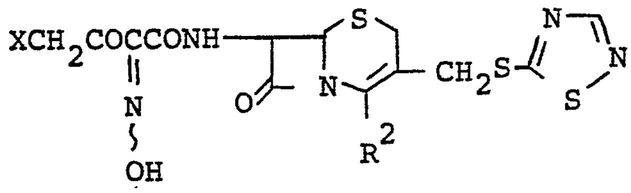

(IVa)

or a salt thereof

wherein $R^2$, A and X are each as defined above.

In the above and subsequent descriptions of the present specification, suitable examples and illustrations of the various definitions which the present invention includes within the scope thereof are explained in detail as follows.

The term "lower" is intended to mean 1 to 6 carbon atom(s), preferably 1 to 4 carbon atom(s), unless otherwise indicated.

The term "higher" is intended to mean 7 to 20 carbon atoms, unless otherwise indicated.

Suitable "protected amino" group may include an amino group substituted by a conventional amino-protective group which is used in penicillin and cephalosporin compounds, for example, acyl as mentioned below, ar(lower)alkyl such as mono-(or di or tri)phenyl(lower)-alkyl (e.g. benzyl, benzhydryl, trityl, etc.), lower alkoxycarbonyl(lower)alkylidene or its enamine tautomer (e.g. 1-methoxycarbonyl-1-propen-2-yl, etc.), di(lower)alkylaminomethylene (e.g. dimethylaminomethylene, etc.), etc.

Suitable "acyl" may include an aliphatic acyl, an aromatic acyl, a heterocyclic acyl and an aliphatic acyl substituted with aromatic or heterocyclic group(s).

The aliphatic acyl may include saturated or unsaturated, acyclic or cyclic ones, such as lower alkanoyl (e.g. formyl, acetyl, propionyl, butyryl, isobutyryl, valeryl, isovaleryl, pivaloyl, hexanoyl, etc.), lower alkanesulfonyl (e.g. mesyl, ethanesulfonyl, propanesulfonyl, etc.), lower alkoxycarbonyl (e.g. methoxycarbonyl, ethoxycarbonyl, propoxycarbonyl, butoxycarbonyl, tert-butoxycarbonyl, etc.), lower alkenoyl (e.g. acryloyl, methacryloyl, crotonoyl, etc.), $(C_3-C_7)$-cycloalkanecarbonyl (e.g. cyclohexanecarbonyl, etc.), amidino, and the like.

The aromatic acyl may include aroyl (e.g. benzoyl, toluoyl, xyloyl, etc.), arenesulfonyl (e.g. benzenesulfonyl, tosyl, etc.), and the like.

The heterocyclic acyl may include heterocycle-carbonyl (e.g. furoyl, thenoyl, nicotinoyl, isonicotinoyl, thiazolylcarbonyl, thiadiazolylcarbonyl, tetrazolyl-carbonyl, etc.), and the like.

The aliphatic acyl substituted with aromatic group(s) may include ar(lower)alkanoyl such as phenyl(lower)alkanoyl (e.g. phenylacetyl, phenylpropionyl, phenylhexanoyl, etc.), ar(lower)alkoxycarbonyl such as phenyl(lower)alkoxycarbonyl (e.g. benzyloxycarbonyl, phenethyloxycarbonyl, etc.), phenoxy(lower)alkanoyl (e.g. phenoxyacetyl, phenoxypropionyl, etc.), and the like.

The aliphatic acyl substituted with heterocyclic group(s) may include thienylacetyl, imidazolylacetyl, furylacetyl, tetrazolylacetyl, thiazolylacetyl, thiadiazolylacetyl, thienylpropionyl, thiadiazolyl-propionyl, and the like.

These acyl groups may be further substituted with one or more suitable substituents such as lower alkyl (e.g. methyl, ethyl, propyl, isopropyl, butyl, pentyl, hexyl, etc.), halogen (e.g. chlorine, bromine, iodine,

fluorine), lower alkoxy (e.g. methoxy, ethoxy, propoxy, isopropoxy, butoxy, pentyloxy, hexyloxy, etc.), lower alkylthio (e.g. methylthio, ethylthio, propylthio, isopropylthio, butylthio, pentylthio, hexylthio, etc.), nitro and the like, and preferable acyl having such substituent(s) may be mono (or di or tri)halo(lower)-alkanoyl (e.g. chloroacetyl, bromoacetyl, dichloroacetyl, trifluoroacetyl, etc.), mono (or di or tri)halo(lower)-alkoxycarbonyl (e.g. chloromethoxycarbonyl, dichloromethoxycarbonyl, 2,2,2-tri-chloroethoxycarbonyl, etc.), nitro (or halo or lower alkoxy)phenyl(lower)alkoxycarbonyl (e.g. nitrobenzyloxycarbonyl, chlorobenzyloxycarbonyl, methoxybenzyloxycarbonyl, etc.), and the like.

Suitable "protected carboxy" may include an esterified carboxy group which is conventionally used in penicillin or cephalosporin compound.

Suitable "ester moiety" in "esterified carboxy" may include lower alkyl ester (e.g. methyl ester, ethyl ester, propyl ester, isopropyl ester, butyl ester, isobutyl ester, t-butyl ester, pentyl ester, tert-pentyl ester, hexyl ester, etc.), lower alkenyl ester (e.g. vinyl ester, allyl ester, etc.), lower alkynyl ester (e.g. ethynyl ester, propynyl ester, etc.), lower alkoxy(lower)alkyl ester (e.g. methoxymethyl ester, ethoxymethyl ester, isopropoxymethyl ester, 1-methoxyethyl ester, 1-ethoxyethyl ester, etc.), lower alkylthio(lower)alkyl ester (e.g. methylthiomethyl ester, ethylthiomethyl ester, ethylthioethyl ester, isopropylthiomethyl ester, etc.), carboxy-substituted-lower alkyl ester (e.g. carboxymethyl ester, 2-carboxyethyl ester, 3-carboxypropyl ester, etc.), protected carboxy-substituted-lower alkyl ester such as lower alkoxycarbonyl-substituted-lower alkyl ester (e.g. tert-butoxycarbonylmethyl ester, 2-tert-butoxycarbonylethyl ester, 3-tert-butoxycarbonylpropyl ester,

etc.), mono(or di or tri)halo(lower)alkyl ester (e.g. 2-iodoethyl ester, 2,2,2-trichloroethyl ester, etc.), lower alkanoyloxy(lower)alkyl ester [e.g. acetoxymethyl ester, propionyloxymethyl ester, butyryloxymethyl ester, valeryloxymethyl ester, pivaloyloxymethyl ester, hexanoyloxymethyl ester, 1(or 2)-acetoxyethyl ester, 1(or 2 or 3)-acetoxypropyl ester, 1(or 2 or 3 or 4)-acetoxybutyl ester, 1(or 2)-propionyloxyethyl ester, 1(or 2 or 3)-propionyloxypropyl ester, 1(or 2)-butyryloxyethyl ester, 1(or 2)-isobutyryloxyethyl ester, 1(or 2)-pivaloyloxyethyl ester, 1(or 2)-hexanoyloxyethyl ester, isobutyryloxymethyl ester, 2-ethylbutyryloxymethyl ester, 3,3-dimethylbutyryloxymethyl ester, 1(or 2)-pentanoyloxyethyl ester, etc.], higher alkanoyloxy(lower)-alkyl ester [e.g. heptanoyloxymethyl ester, octanoyloxymethyl ester, nonanoyloxymethyl ester, decanoyloxymethyl ester, undecanoyloxymethyl ester, lauroyloxymethyl ester, tridecanoyloxymethyl ester, myristoyloxymethyl ester, pentadecanoyloxymethyl ester, palmitoyloxymethyl ester, heptadecanoyloxymethyl ester, stearoyloxymethyl ester, nonadecanoyloxymethyl ester, eicosanoyloxymethyl ester, 1(or 2)-heptanoyloxyethyl ester, 1(or 2)-octanoyloxyethyl ester, 1(or 2)-nonanoyloxyethyl ester, 1(or 2)-decanoyloxyethyl ester, 1(or 2)-undecanoyloxyethyl ester, 1(or 2)-lauroyloxyethyl ester, 1(or 2)-tridecanoyloxyethyl ester, 1(or 2)-myristoyloxyethyl ester, 1(or 2)-pentadecanoyloxy ethyl ester, 1(or 2)-palmitoyloxyethyl ester, 1(or 2)-heptadecanoyloxyethyl ester, 1(or 2)-stearoyloxy-ethyl ester, 1(or 2)-nonadecanoyloxyethyl ester, 1(or 2)-eicosanoyloxyethyl ester, etc.], lower alkoxycarbonyloxy-(lower)alkyl ester [e.g. methoxycarbonyloxymethyl ester, ethoxycarbonyloxymethyl ester, propoxycarbonyloxymethyl ester, isopropyloxycarbonyloxymethyl ester, tert-butoxy-

carbonyloxymethyl ester, 1(or 2)-methoxycarbonyloxyethyl ester, 1(or 2)-ethoxycarbonyloxyethyl ester, 1(or 2)-propoxycarbonyloxyethyl ester, 1(or 2)-isopropoxycarbonyloxyethyl ester, 1(or 2)-butoxycarbonyloxyethyl ester, 1(or 2)-isobutoxycarbonyloxy-ethyl ester, 1(or 2)-tert-butoxycarbonyloxyethyl ester, 1(or 2)-hexyloxycarbonyloxyethyl ester, 1(or 2 or 3)-methoxycarbonyloxypropyl ester, 1(or 2 or 3)-ethoxycarbonyloxypropyl ester, 1(or 2 or 3)-isopropoxycarbonyloxypropyl ester, 1(or 2 or 3 or 4)-ethoxycarbonyloxybutyl ester, 1(or 2 or 3 or 4)-butoxycarbonyloxybutyl ester, 1(or 2 or 3 or 4 or 5)-pentyloxycarbonyloxypentyl ester, 1(or 2 or 3 or 4 or 5)-neopentyloxycarbonyloxypentyl ester, 1(or 2 or 3 or 4 or 5 or 6)-ethoxycarbonyloxyhexyl ester, etc.], (5-lower alkyl-2-oxo-1,3-dioxol-4-yl)(lower)alkyl ester [e.g. (5-methyl-2-oxo-1,3-dioxol-4-yl)methyl ester, (5-ethyl-2-oxo-1,3-dioxol-4-yl)methyl ester, (5-propyl 2-oxo-1,3-dioxol-4-yl)ethyl ester, etc.], lower alkanesulfonyl(lower)alkyl ester (e.g. mesylmethyl ester, 2-mesylethyl ester, etc.), ar(lower)alkyl ester which may have one or more substituent(s) such as mono-(or di or tri)phenyl(lower)alkyl ester which may have one or more suitable substituent(s) (e.g. benzyl ester, 4-methoxybenzyl ester, 4-nitrobenzyl ester, phenethyl ester, benzhydryl ester, trityl ester, bis(methoxyphenyl)-methyl ester, 3,4-dimethoxybenzyl ester, 4-hydroxy-3,5-di-t-butylbenzyl ester, etc.), aryl ester which may have one or more suitable substituents (e.g. phenyl ester, tolyl ester, t-butylphenyl ester, xylyl ester, mesityl ester, cumenyl ester, salicyl ester, etc.), heterocyclic ester (e.g. phthalidyl ester, etc.), and the like.

Suitable "acid residue" may include halogen (e.g. fluorine, chlorine, bromine, iodine, etc.), acyloxy in which the acyl moiety can be referred to one as aforementioned, and the like.

Suitable "esterified carboxy" can be referred to one for the protected carboxy as mentioned above.

Suitable "hydroxy-protective group" may include aforesaid acyl, ar(lower)alkyl (e.g. benzyl, trityl, etc.), lower alkoxy(lower)alkyl (e.g. 1-methyl-1-methoxyethyl, methoxypropyl, etc.), tetrahydropyranyl, and the like.

Suitable "a group which is replaceable with the formula :

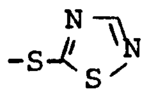

" may include acid residue as aforementioned such as halogen, acyloxy, for example, lower alkanoyloxy (e.g. acetoxy, etc.).

Preferred embodiments of the terms $R^1$, $R^2$ and $R^3$ of the object compound (I) are as follows.

The term "$R^1$" is amino or ar(lower)alkylamino such as mono(or di or tri)phenyl(lower)alkylamino, more preferably amino or triphenyl($C_1$-$C_4$)alkylamino, the most preferably amino or tritylamino;

The term "$R^2$" is carboxy or esterified carboxy such as lower alkanoyloxy(lower)alkoxycarbonyl and ar(lower)-alkoxycarbonyl which may be substituted by nitro, more preferably carboxy, $C_1$-$C_4$alkanoyloxy($C_1$-$C_4$)alkoxycarbonyl, nitrophenyl($C_1$-$C_4$)alkoxycarbonyl or diphenyl($C_1$-$C_4$)-alkoxycarbonyl, and the most preferably carboxy, pivaloyloxymethoxycarbonyl, nitrobenzyloxycarbonyl or benzhydryloxycarbonyl.

The term "$R^3$" is hydrogen, tetrahydropyranyl or acyl such as lower alkanoyl, more preferably hydrogen, tetrahydropyran-2-yl or $C_1$-$C_4$alkanoyl, and the most preferably hydrogen, tetrahydropyran-2-yl or acetyl.

The processes for preparing the object compound of the present invention are explained in detail in the following.

<u>Process 1</u> :

The object compound (I) or a salt thereof can be

prepared by reacting the compound (II) or its reactive derivative at the amino group or a salt thereof with the compound (III) or its reactive derivative at the carboxy group or a salt thereof.

Suitable reactive derivative at the amino group of the compound (II) may include Schiff's base type imino or its tautomeric enamine type isomer formed by the reaction of the compound (II) with a carbonyl compound such as aldehyde, ketone or the like; a silyl derivative formed by the reaction of the compound (II) with a silyl compound such as bis(trimethylsilyl) acetamide, mono(trimethylsilyl)acetamide, N,N-bis-(trimethylsilyl)urea, or the like;
a derivative formed by reaction of the compound (II) with phosphorus trichloride or phosgene, and the like.

Suitable salt of the compounds (II) and (III) may include an acid addition salt such as an organic acid salt (e.g. acetate, maleate, tartrate, benzenesulfonate, toluenesulfonate, etc.) or an inorganic acid salt (e.g. hydrochloride, hydrobromide, sulfate, phosphate, etc.); a metal salt (e.g. sodium salt, potassium salt, calcium salt, magnesium salt, etc.); ammonium salt; an organic amine salt (e.g. triethylamine salt, dicyclohexylamine salt, etc.), and the like.

Suitable reactive derivative at the carboxy group of the compound (III) may include an acid halide, an acid anhydride, an activated amide, an activated ester, and the like. The suitable example may be an acid chloride, an acid azide; a mixed acid anhydride with an acid such as substituted phosphoric acid (e.g. dialkylphosphoric acid, phenylphosphoric acid, diphenylphosphoric acid, dibenzylphosphoric acid, halogenated phosphoric acid, etc.), dialkylphosphorous acid, sulforous acid, thiosulfuric acid, sulfuric acid, alkylcarbonic acid, aliphatic carboxylic acid (e.g.

pivalic acid, pentanoic acid, isopentanoic acid, 2-ethylbutyric acid or trichloroacetic acid, etc.) or aromatic carboxylic acid (e.g. benzoic acid, etc.); a symmetrical acid anhydride; an activated amide with imidazole, 4-substituted imidazole, dimethylpyrazole, triazole or tetrazole; or an activated ester (e.g. cyanomethyl ester, methoxymethyl ester, dimethyliminomethyl $[(CH_3)_2\overset{+}{N}=CH-]$ ester, vinyl ester, propargyl ester, p-nitrophenyl ester, 2,4-dinitrophenyl ester, trichlorophenyl ester, pentachlorophenyl ester, mesylphenyl ester, phenylazophenyl ester, phenyl thioester, p-nitrophenyl thioester, p-cresyl thioester, carboxymethyl thioester, pyranyl ester, pyridyl ester, piperidyl ester, 8-quinolyl thioester, etc.), or an ester with a N-hydroxy compound (e.g. N,N-dimethylhydroxylamine, 1-hydroxy-2-(1H)-pyridone, N-hydroxysuccinimide, N-hydroxyphthalimide, 1-hydroxy-1H-benzotriazole, 1-hydroxy-6-chloro-1H-benzotriazole, etc.), and the like. These reactive derivatives can optionally be selected from them according to the kind of the compound (III) to be used.

The reaction is usually carried out in a conventional solvent such as water, acetone, dioxane, acetonitrile, chloroform, dichloromethane, ethylene chloride, tetrahydrofuran, ethyl acetate, N,N-dimethylformamide, N,N-dimethylacetamide, pyridine or any other organic solvent which does not adversely influence the reaction. These conventional solvents may also be used in a mixture with water.

When the compound (III) is used in free acid form or its salt form in the reaction, the reaction is preferably carried out in the presence of a conventional condensing agent such as N,N'-dicyclohexylcarbodiimide; N-cyclohexyl-N'-morpholinoethylcarbodiimide; N-cyclohexyl-N'-(4-diethylaminocyclohexyl)carbodiimide;

N,N'-diethylcarbodiimide, N,N'-diisopropylcarbodiimide; N-ethyl-N'-(3-dimethylaminopropyl)carbodiimide; N,N-carbonylbis-(2-methylimidazole); pentamethyleneketene-N-cyclohexylimine; diphenylketene-N-cyclohexylimine; ethoxyacetylene; 1-alkoxy-1-chloroethylene; 1,1'-(carbonyldioxy)-dibenzotriazole, 1,1'-dibenzotriazolyloxallate trialkyl phosphite; ethyl polyphosphate; isopropyl polyphosphate; phosphorus oxychloride (phosphoryl chloride); phosphorus trichloride; thionyl chloride; oxalyl chloride; triphenylphosphine; 2-ethyl-7-hydroxybenzisoxazolium salt; 2-ethyl-5-(m-sulfophenyl)isoxazolium hydroxide intramolecular salt; 1-(p-chlorobenzenesulfonyloxy)-6-chloro-1H-benzotriazole; so-called Vilsmeier reagent prepared by the reaction of dimethylformamide with thionyl chloride, phosgene, phosphorus oxychloride, etc.; or the like.

The reaction may also be carried out in the presence of an inorganic or organic base such as an alkali metal bicarbonate, tri(lower)alkylamine, pyridine, N-(lower)alkylmorphorine, N,N-di(lower)alkylbenzylamine, or the like. The reaction temperature is not critical, and the reaction is usually carried out under cooling to warming.

The present reaction includes, within its scope, the case that the hydroxy-protective group for $R^3$ is eliminated during the reaction or the post-treating step of the present process.

<u>Process 2</u> :

The compound (Ib) or a salt thereof can be prepared by subjecting the compound (Ia) or a slat thereof to elimination reaction of the carboxy-protective group.

Suitable salts of the compounds (Ia) and (Ib) may include the same ones as exemplified for the compound (I).

Suitable method for this elimination reaction may include conventional one such as hydrolysis, reduction, or the like.

(i) For hydrolysis :

Hydrolysis is preferably carried out in the presence of an acid.

Suitable acid may be an inorganic acid (e.g. hydrochloric acid, hydrobromic acid, sulfuric acid, etc.), an organic acid (e.g. formic acid, acetic acid, trifluoroacetic acid, propionic acid, methanesulfonic acid, benzenesulfonic acid, p-toluenesulfonic acid, etc.), an acidic ion-exchange resin and the like. In case that the organic acid such as trifluoroacetic acid and p-toluenesulfonic acid is used in this reaction, the reaction is preferably carried out in the presence of cation trapping agents (e.g. anisole, etc.).

Further, instead of the above acid, Lewis acid such as boron trifluoride, boron trifluoride etherate, aluminum trichloride, antimony pentachloride, ferric chloride, stannic chloride, titanium tetrachloride, zinc chloride, and the like can also be used in this reaction, and in case of using Lewis acid, the reaction can preferably be carried out in the presence of cation trapping agent (e.g. anisole).

The hydrolysis is usually conducted in the absence or the presence of a conventional solvent which does not adversely influence the reaction such as water, methanol ethanol, propanol, tert-butyl alcohol, tetrahydrofuran, N,N-dimethylformamide, N,N-dimethylacetamide, dioxane, dichloromethane, or a mixture thereof, and further the abovementioned acids can also be used as a solvent when they are in liquid.

The reaction temperature of this hydrolysis is not critical, and the reaction is usually conducted under cooling to at somewhat elevated temperature.

(ii) For Reduction :

Reduction is conducted in a conventional manner, including chemical reduction and catalytic reduction.

Suitable reducing agents to be used in chemical reduction are a combination of a metal (e.g. tin, zinc, iron, etc.) or metallic compound (e.g. chromium chloride, chromium acetate, etc.) and an organic or inorganic acid (e.g. formic acid, acetic acid, propionic acid, trifluoroacetic acid, p-toluenesulfonic acid, hydrochloric acid, hydrobromic acid, etc.).

Suitable catalysts to be used in catalytic reduction are conventional ones such as platinum catalysts (e.g. platinum plate, spongy platinum, platinum black, colloidal platinum, platinum oxide, platinum wire, etc.), palladium catalysts (e.g. spongy palladium, palladium black, palladium oxide, palladium on carbon, colloidal palladium, palladium on barium sulfate, palladium on barium carbonate, etc.), nickel catalysts (e.g. reduced nickel, nickel oxide, Raney nickel, etc.), cobalt catalysts (e.g. reduced cobalt, Raney cobalt, etc.), iron catalysts (e.g. reduced iron, Raney iron, etc.), copper catalysts (e.g. reduced copper, Raney copper, Ullman copper, etc.) and the like.

The reduction is usually conducted in a conventional solvent which does not adversely influence the reaction such as water, methanol, ethanol, propanol, N,N-dimethylformamide, or a mixture thereof. Additionally, in case that the abovementioned acids to be used in chemical reduction are in liquid, they can also be used as a solvent. Further, a suitable solvent to be used in catalytic reduction may be the abovementioned solvent, and other conventional solvent such as diethyl ether, dioxane, tetrahydrofuran, etc., or a mixture thereof.

The reaction temperature of this reduction is not

critical and the reaction is usually conducted under cooling to warming.

The present elimination reaction of the carboxy protective group includes, within its scope, the cases that protected amino group for $R^1$ is converted into the free amino group or the hydroxy-protective group for $R^3$ is eliminated at the same time during the reaction or the post-treating step of the present process.

Process 3 :

The compound (I) or a salt thereof can be prepared by reacting the compound (IV) or a salt thereof with the compound (V).

Suitable salt of the compound (IV) may include the same salt as exemplified for the compound (I).

This reaction is usually conducted in a conventional solvent which does not adversely influence the reaction such as ethyl acetate, dichloromethane, chloroform, carbon tetrachloride, tetrahydrofuran, N,N-dimethylformamide, N,N-dimethylacetamide, dioxane, water, acetic acid, formic acid, etc. or a mixture thereof.

The reaction temperature is not critical and the reaction is usually conducted under cooling to warming.

Process 4 :

The compound (Ic) or a salt thereof can be prepared by subjecting the compound (Ib) or a salt thereof to esterification.

Suitable salt of the compounds (Ib) and (Ic) can be referred to the ones as exemplified for the compound (I).

The present reaction may be carried out by reacting the compound (Ib) or a salt thereof with esterifying agent.

Suitable esterifying agent may be a compound of the formula :

$$Z-R^7$$

wherein $R^7$ is ester moiety of esterified carboxy as mentioned above, and

Z is hydroxy or its reactive derivative.

Suitable reactive derivative of hydroxy for Z may include an acid residue such as aforesaid halogen, acyloxy, or the like.

The present reaction is usually carried out in a solvent such as dimethylformamide, pyridine, hexamethylphosphoric triamide, dimethylsulfoxide or any other solvent which does not adversely affect the reaction.

In case that the compound (Ib) is used in a form of free acid, the reaction is preferably carried out in the presence of a base or condensing agent as mentioned in Process 1.

The reaction temperature is not critical and the reaction is preferably carried out under cooling, at ambient temperature or under warming.

Process 5 :

The compound (Ie) or a salt thereof can be prepared by subjecting the compound (Id) or a salt thereof to elimination reaction of the hydroxy-protective group.

Suitable salt of the compounds (Id) and (Ie) may include the same ones as exemplified for the compound (I).

This elimination reaction of the hydroxy-protective group of the compound (Id) can be carried out in a similar manner to that of the aforementioned Process 2, and therefore the reagents to be used and the reaction conditions (e.g. solvent, reaction temperature, etc.) are referred to those of the Process 2.

The present elimination reaction includes, within its scope, the case that the protected amino for $R^1$ and/or protected carboxy group for $R^2$ are converted into the corresponding free amino and/or free carboxy group during the reaction or the post-treating step of the present process.

Process 6 :

The compound (Ig) or a salt thereof can be prepared by subjecting the compound (If) or a salt thereof to elimination reaction of the amino-protective group.

Suitable salt of the compounds (If) and (Ig) may include the same ones as exemplified for the compound (I).

This elimination reaction can be carried out in a similar manner to that of the aforementioned Process 2, and therefore the reagents to be used and the reaction conditions (e.g. solvent, reaction temperature, etc.) are referred to those of the Process 2.

The present elimination reaction includes, within its scope, the case that the protected carboxy group for $R^2$ is converted into the free carboxy group and the hydroxy-protective group for $R^3$ is eliminated at the same during the reaction or the post-treating step of the present process.

Process 7 :

The object compound (I) or a salt thereof can be prepared by reacting a compound (VI) or a salt thereof with a compound (IX) or its reactive derivative.

Suitable salt of the compound (VI) may include the same ones exemplified for the compound (I).

Suitable reactive derivative at the mercapto group of the compound (IX) may include a metal salt such as

an alkali metal salt (e.g. sodium salt, potassium salt, etc.), an alkaline earth metal salt (e.g. magnesium salt, etc.) or the like.

The reaction can be carried out in the presence of sodium iodide, sodium thiocyanate and the like.

The reaction is usually carried out in a solvent such as water, acetone, chloroform, nitrobenzene, dichloromethane, ethylene chloride, dimethylformamide, methanol, ethanol, ether, tetrahydrofuran or any other conventional solvents which do not adversely influence the reaction, preferably in ones having strong polarity, which may be used as a mixture with water.

When the compound (VI) and/or the compound (IX) are used in free form in the reactions, the reaction is preferably carried out in the presence of a base, for example, an organic or an inorganic base such as alkali metal hydroxide, alkali metal carbonate, alkali metal bicarbonate, trialkylamine, pyridine or the like, and preferably carried out around neutral conditions. The reaction temperature is not critical and the reaction is usually carried out at ambient temperature or under heating.

The processes for preparing the starting compounds of the present invention are explained in detail in the following.

Process ① :

The compound (VIII) or a salt thereof can be produced by reacting the compound (II) or a reactive derivative at the amino group thereof, or a salt thereof with the compound (VII) or a reactive derivative at the carboxy group thereof or a salt thereof.

Suitable reactive derivative of the compound (VII) may include an acid halide such as acid chloride,

acid bromide, or the like, which can be prepared, for example, by the reaction of diketene and halogen.

Suitable salts of the compound (VII) may include the same salt with a base as exemplified for the compound (I), and suitable salts of the compound (VIII) may include the same ones for the compound (I).

The reaction is usually conducted in a conventional solvent which does not adversely influence the reaction such as water, acetone, dioxane, acetonitrile, chloroform, benzene, dichloromethane, ethylene chloride, tetrahydrofuran, ethyl acetate, N,N-dimethylformamide, pyridine, hexamethylphosphoramide, etc., or a mixture thereof.

The reaction temperature is not critical and the reaction is usually conducted under cooling to warming.

Process (2) :

The compound (IVa) or a salt thereof can be produced by reacting the compound (VIII) or a salt thereof with a nitrosating agent.

Suitable salts of the compound (IVa) may include the same ones as exemplified for the compound (I).

Suitable nitrosating agent may include nitrous acid and its conventional derivatives such as nitrosyl halide (e.g. nitrosyl chloride, nitrosyl bromide, etc.), alkali metal nitrite (e.g. sodium nitrite, potassium nitrite, etc.), alkyl nitrite (e.g. butyl nitrite, pentyl nitrite, isoamyl nitrite, etc.), and the like.

In case that a salt of nitrous acid or its alkali metal salt is used as a nitrosating agent, the reaction is preferably carried out in the presence of an acid such as an inorganic or organic acid (e.g. hydrochloric

acid, sulfuric acid, formic acid, acetic acid, etc.).

This reaction can preferably be carried out in the presence of acyl chloride (e.g. acetyl chloride) or an activated methylene compound such as acetylacetone, ethyl acetoacetate, and the like.

This reaction is usually conducted in a conventional solvent which does not adversely influence the reaction such as water, acetic acid, benzene, methanol, ethanol, tetrahydrofuran, dichloromethane, or a mixture thereof. The reaction temperature is not critical and the reaction is preferably conducted within the range of cooling to warming.

The compound (IVa) of this reaction may include syn isomer, anti isomer and a mixture thereof at the hydroxyimino group thereof, and such compound may be represented by the partial formula :

-C-<br>||<br>N<br>~<br>OH .

The object compounds (I), (Ib), (Ic), (Ie) and (Ig) obtained in the Processes 1 to 7, and the compounds (IVa) and (VIII) obtained in the Processes ① and ② can be isolated and purified in a conventional manner, for example, extraction, precipitation, fractional crystallization, recrystallization, chromatography, and the like.

With respect to the object compound (I), in case that it possesses a free amino group for $R^1$ and a free carboxy group for $R^2$, it may be transformed into its pharmaceutically acceptable salts by a conventional method.

The object compound (I) and the pharmaceutically acceptable salt thereof of the present invention are novel and exhibit high antimicrobial activity, inhibiting the growth of a wide variety of pathogenic microorganisms including Gram-positive and Gram-netative microorganisms, especially Gram-positive bacteria such as Staphylococcus aureus and are useful as antimicrobial agents.

In order to illustrate the usefulness of the object compound, anti-microbial activities of representative compound of the present invention are shown below as compared to the known compounds.

Minimal inhibitory concentrations

(A) Test Method

In vitro antimicrobial activity was determined by the two-fold agar-plate dilution method as described below.

One loopful of an overnight culture of each test strain in Trypticase-soy broth ($10^8$ viable cells per ml) was streaked on heart infusion agar (HI-agar) containing graded concentrations of representative test compound, and the minimal inhibitory concentration (MIC) was expressed in terms of μg/mℓ after incubation at 37°C for 20 hours.

(B) Test Compounds

7-[2-(2-Aminothiazol-4-yl)-2-hydroxyiminoacetamido]-3-(1,2,4-thiadiazol-5-yl)thiomethyl-3-cephem-4-carboxylic acid (syn isomer) [hereinafter referred to as Compound (1)].

7-[2-(2-Aminothiazol-4-yl)-2-hydroxyiminoacetamido]-3-(3-methyl-1,2,4-thiadiazol-5-yl)thiomethyl-3-cephem-4-carboxylic acid (syn isomer) [reference compound (A)] [hereinafter referred to as Compound (A)]

Cefotaxime sodium [sodium salt of reference Compound (B)] [hereinafter referred to as Compound (B]

Cefmenoxime [reference compound (C)] [hereinafter referred to as Compound (C)]

Cefotiam [reference compound (D)] [hereinafter referred to as Compound (D)]

(C) Test Results

M.I.C. (μg/mℓ)

| Compounds / Test strains | (1) | (A) | (B) | (C) | (D) |
|---|---|---|---|---|---|
| Staphylococcus aureus 32 | 0.39 | 1.56 | 3.13 | 3.13 | 1.56 |
| Staphylococcus aureus 3004 | 6.25 | 50 | >100 | >100 | >100 |
| Escherichia coli 29 | <0.025 | 0.10 | 0.05 | 0.05 | 0.39 |

For therapeutic administration, the object compound (I) and the pharmaceutically acceptable salt thereof of the present invention are used in the form of conventional

pharmaceutical preparation which contains said compound as an active ingredient, in admixture with pharmaceutically acceptable carriers such as an organic or inorganic solid or liquid excipient which is suitable for oral, parenteral and external administration. The pharmaceutical preparations may be in solid form such as tablet, granule, powder, capsule, or liquid form such as solution, suspension, syrup, emulsion, lemonade and the like.

If needed, there may be included in the above preparations auxiliary substances, stabilizing agents, wetting agents and other commonly used additives such as lactose, citric acid, tartaric acid, stearic acid, magnesium stearate, terra alba, sucrose, corn starch, talc, gelatin, agar, pectin, peanut oil, olive oil, cacao butter, ethylene glycol, and the like.

While the dosage of the compound (I) may vary from and also depend upon the age, conditions of the patient, a kind of diseases, a kind of the compound (I) to be applied, etc. In general amounts between 1 mg and about 4,000 mg or even more per day may be administered to a patient. An average single dose of about 50 mg, 100 mg, 250 mg, 500 mg, 1000 mg, 2000 mg of the object compound (I) of the present invention may be used in treating diseases infected by pathogenic microorganisms.

The following Preparations and Examples are given for the purpose of illustrating the present invention.

Preparation 1

To a solution of 7-amino-3-(1,2,4-thiadiazol-5-yl)-thiomethyl-3-cephem-4-carboxylic acid (3.5 g) and N,N'-bis(trimethylsilyl)urea (6.5 g) in tetrahydrofuran (100 ml) was added 4-bromoacetoacetyl bromide obtained from diketene (1.15 g) and bromine (2.15 g) in dichloromethane (7.5 ml) under cooling at -20°C and

the mixture was stirred for 30 minutes at -15°C. The reaction mixture was poured into a mixture of ethyl acetate (100 ml) and water (150 ml). The organic layer was separated, washed with water and brine, and evaporated. The residue was pulverized in diisopropyl ether (300 ml). The resultant precipitate was collected by filtration and dried in vacuo to give 7-(4-bromoacetoacetamido)-3-(1,2,4-thiadiazol-5-yl)thiomethyl-3-cephem-4-carboxylic acid (4.64 g).

NMR (DMSO-$d_6$, δ) : 3.90-4.00 (4H, m), 4.20 (2H, ABq, J=12Hz), 4.50 (2H, s), 5.20 (1H, d, J=5Hz), 5.73 (1H, d-d, J=5Hz, 8Hz), 8.70 (1H, s), 9.10 (1H, d, J=8Hz)

Preparation 2

The following compound was obtained according to a similar manner to that of Preparation 1.

Benzhydryl 7-(4-chloroacetoacetamido)-3-(1,2,4-thiadiazol-5-yl)thiomethyl-3-cephem-4-carboxylate.

IR (Nujol) : 1775, 1710 $cm^{-1}$

NMR (DMSO-$d_6$, δ) : 3.40-3.93 (4H, m), 4.18 and 4.63 (2H, ABq, J=14Hz), 4.55 (2H, s), 5.16 (1H, d, J=5Hz), 5.84 (1H, d-d, J=5Hz, 8Hz), 7.01 (1H, s), 7.28 (1H, s), 7.13-7.67 (10H, m), 8.52 (1H, s), 9.04 (1H, d, J=8Hz)

Preparation 3

(1) To a solution of benzhydryl 2-(2-tritylaminothiazol-4-yl)-2-hydroxyiminoacetate (syn isomer) (2.8 g) in dichloromethane (56 ml) was added pyridine (0.47 ml) under ice-cooling. Then a solution of acetyl chloride (0.47 ml) in dichloromethane (5.6 ml) was added thereto. After stirring for 20 minutes under ice-cooling, the reaction mixture was washed with

water and dried over magnesium sulfate. The solvent was distilled off and the residue was pulverized in a mixture of diethyl ether and n-hexane. The resultant precipitate was collected by filtration to give benzhydryl 2-(2-tritylaminothiazol-4-yl)-2-acetoxyiminoacetate (syn isomer) (3.0 g).

IR (Nujol) : 3310, 1750, 1600, 1530 $cm^{-1}$

NMR ($DMSO-d_6$, δ) : 1.88 (3H, s), 6.75 (1H, s), 7.07-7.57 (26H, m), 8.87 (1H, s)

(2) To a solution of benzhydryl 2-(2-tritylaminothiazol-4-yl)-2-acetoxyiminoacetate (syn isomer) (3.0 g) in a mixture of dichloromethane (60 ml) and anisole (1.5 ml) was added trifluoroacetic acid (5 ml) under ice-cooling. The mixture was stirred for 4 hours to give crystals, which were collected by filtration and washed with diisopropyl ether and water to give 2-(2-aminothiazol-4-yl)-2-acetoxyiminoacetic acid (syn isomer) (0.5 g).

NMR ($DMSO-d_6$, δ) : 1.48 (3H, s), 7.22 (1H, s), 7.13-7.47 (2H, m)

Preparation 4

To a solution of benzhydryl 7-(4-chloroacetoacetamido)-3-(1,2,4-thiadiazol-5-yl)thiomethyl-3-cephem-4-carboxylate (720 g) in dichloromethane (13 ℓ) were added isoamylnitrite (176 g) and acetyl chloride (91.8 g) at room temperature, and the mixture was stirred for 1 hour at the same temperature. The reaction mixture was poured into a mixture of tetrahydrofuran (3.2 ℓ) and a saturated sodium chloride aqueous solution (7.2 ℓ). The organic layer was separated, washed with brine (7.2 ℓ) and then dried. The solution was evaporated, and the residue was pulverized in n-hexan (36 ℓ). The precipitates were collected by filtration, washed with n-hexan and

dried over phosphorus pentoxide to give benzhydryl 7-(4-chloro-2-hydroxyiminoacetoacetamido)-3-(1,2,4-thiadiazol-5-yl)thiomethyl-3-cephem-4-carboxylate (688.6 g).

IR (Nujol) : 1780, 1710 $cm^{-1}$

NMR (DMSO-$d_6$, δ) : 3.57 and 3.8 (2H, ABq, J=18Hz), 4.68 and 4.22 (2H, ABq, J=14Hz), 4.76 (2H, s), 5.06 (1H, d, J=5Hz), 5.86 (1H, d-d, J=5Hz, 8Hz), 7.02 (1H, s), 7.28 (1H, s), 7.17-7.63 (10H, m), 8.31 (1H, s), 9.40 (1H, d, J=8Hz), 11.29 (1H, s)

Example 1

To a solution of 7-(4-bromoacetoacetamido)-3-(1,2,4-thiadiazol-5-yl)thiomethyl-3-cephem-4-carboxylic acid (4.64 g) in a mixture of dichloromethane (40 ml) and acetic acid (20 ml) was dropwise added an aqueous solution (4.3 ml) of sodium nitrite (0.84 g) at -10°C. After stirring for 1.5 hours, the reaction mixture was poured into a mixture of water (80 ml) and tetrahydrofuran (40 ml). The separated organic layer was washed with water and brine. The solvent was distilled off to give 7-(4-bromo-2-hydroxyiminoacetoacetamido)-3-(1,2,4-thiadiazol-5-yl)thiomethyl-3-cephem-4-carboxylic acid. To this product was added a solution of thiourea (0.86 g) in dimethylacetamide (13 ml). The mixture was stirred for 2 hours at ambient temperature. The reaction mixture was poured into a mixture of ethyl acetate (50 ml) and water (70 ml). The mixture was adjusted to pH 6.5 with 2N aqueous sodium hydroxide. The separated aqueous layer was concentrated and adjusted to pH 4.4 with 1N hydrochloric acid. The acidified aqueous solution was passed through a column of a non-ionic adsorption resin "Diaion HP-20" (Trademark : prepared by Mitsubishi Chemical Industries), eluted with 20% aqueous isopropyl alcohol. The eluate was lyophilized to give 7-[2-(2-aminothiazol-4-yl)-2-hydroxyiminoacetamido]-3-(1,2,4-thiadiazol-5-yl)thiomethyl-3-cephem-4-carboxylic acid (syn isomer) (0.70 g).

IR (Nujol) : 2900, 1770, 1620, 1520 $cm^{-1}$

NMR ($DMSO-d_6$, δ) : 3.63 (2H, ABq, J=18Hz), 4.42 (2H, ABq, J=13Hz), 5.12 (1H, d, J=5Hz), 5.77 (1H, d-d, J=5Hz, 8Hz), 6.62 (1H, s), 8.68 (1H, s), 9.38 (1H, d, J=8Hz)

Example 2

To a mixture of dimethylformamide (0.38 g) and tetrahydrofuran (1.2 ml) was added phosphorus oxychloride (0.8 g) at 0°C. After stirring for 30 minutes, a solution of 2-(2-tritylaminothiazol-4-yl)-2-(2-tetrahydropyranyloxyimino)acetic acid (syn isomer) (2.07 g) was added thereto. The mixture was stirred for 1 hour at -3 to 3°C to produce an activated acid solution. To a solution of benzhydryl 7-amino-3-(1,2,4-thiadiazol-5-yl)thiomethyl-3-cephem-4-carboxylate (2.0 g) in tetrahydrofuran (40 ml) containing monotrimethylsilylacetamide (4 g) was added at once the above obtained active acid solution at -20°C and the mixture was stirred for 1.5 hours at -15 to -10°C. The reaction mixture was poured into a mixture of ethyl acetate, tetrahydrofuran and water. The separated aqueous layer was extracted with a mixed solvent of ethyl acetate and tetrahydrofuran several times. The combined organic layer was washed with brine and dried over magnesium sulfate. The solvent was evaporated and the residue was pulverized in diisopropyl ether, collected by filtration and dried over phosphorus pentoxide to give benzhydryl 7-[2-(2-tetrahydropyranyloxyimino)-2-(2-tritylaminothiazol-4-yl)acetamido]-3-(1,2,4-thiadiazol-5-yl)-thiomethyl-3-cephem-4-carboxylate (syn isomer) (4.40 g).

IR (Nujol) : 3350, 1770, 1700, 1660 $cm^{-1}$

NMR ($DMSO-d_6$, δ) : 1.50-2.00 (m), 3.50-4.30 (4H, m),

5.30 (1H, d, J=5Hz), 5.85 (1H, dd, J=5Hz, J=8Hz), 6.83 (1H, s), 7.03 (1H, s), 7.10-7.77 (m), 8.57 (1H, s), 9.67 (1H, d, J=8Hz)

Example 3

To a mixture of benzhydryl 7-[2-(2-tetrahydropyranyl-oxyimino)-2-(tritylaminothiazol-4-yl)acetamido]-3-(1,2,4-thiadiazol-5-yl)thiomethyl-3-cephem-4-carboxylate (syn isomer) (4.0 g) and anisole (4 ml) was added trifluoroacetic acid (8 ml). After stirring for two hours at room temperature, the mixture was poured into diisopropyl ether (100 ml). The resulting precipitate was collected by filtration and dissolved in 5% aqueous sodium hydrogen carbonate. The aqueous solution was washed with ethyl acetate adjusted to pH 4.5 with 1N hydrochloric aicd and subjected to column chromatography on a non-ionic adsorption resin "Diaion HP-20". Elution was carried out with 15% aqueous isopropyl alcohol and the eluate containing a desired compound was collected. This eluate was acidified to pH 2.0 with 10% hydrochloric acid under ice-cooling. The resulting precipitate was collected, washed with water and dried to give 7-[2-(2-aminothiazol-4-yl)-2-hydroxy-iminoacetamido]-3-(1,2,4-thiadiazol-5-yl)thiomethyl-3-cephem-4-carboxylic acid (syn isomer) (0.13 g).

IR (Nujol) : 3200, 1750, 1690, 1650 $cm^{-1}$

NMR ($DMSO-d_6$, δ) : 3.52 and 3.78 (2H, ABq, J=18Hz), 4.30 and 4.62 (2H, ABq, J=13Hz), 5.13 (1H, d, J=5Hz), 5.78 (1H, d-d, J=5Hz, 8Hz), 6.65 (1H, s), 7.07 (2H, br s), 8.70 (1H, s), 9.42 (1H, d, J=8Hz), 11.36 (1H, br s)

Example 4

To a solution of benzhydryl 7-(4-chloro-2-hydroxyiminoacetoacetamido)-3-(1,2,4-thiadiazol-5-yl)-

thiomethyl-3-cephem-4-carboxylate (680 g) in dimethylacetamide(2.7 ℓ) was added thiourea (160.8 g) at room temperature, and the mixture was stirred for 3 hours at the same temperature. The reaction mixture was poured into a mixture of ethyl acetate (9.5 ℓ) and ice-water (4.7 ℓ). The separated organic layer was washed with water (4.7 ℓ x 2) and brine (4.7 ℓ), dried over magnesium sulfate and then evaporated. The residue (1.5 ℓ) was pulverized in a mixture of ethyl acetate (4.5 ℓ) and diisopropyl ether (9 ℓ). The precipitates were collected by filtration, washed with diisopropyl ether and dried over phosphorus pentoxide to give benzhydryl 7-[2-(2-aminothiazol-4-yl)-2-hydroxyiminoacetamido]-3-(1,2,4-thiadiazol-5-yl)thiomethyl-3-cephem-4-carboxylate (syn isomer) (436.5 g).

IR (Nujol) : 1780, 1720, 1665, 1620 $cm^{-1}$

NMR ($DMSO-d_6$, δ) : 3.59 and 3.83 (2H, ABq, J=18Hz), 4.23 and 4.58 (2H, ABq, J=13Hz), 5.23 (1H, d, J=5Hz), 5.90 (1H, d-d, J=5Hz, 8Hz), 6.67 (1H, s), 7.00-7.80 (10H, m), 8.55 (1H, s), 9.46 (1H, d, J=8Hz), 11.40 (1H, s)

Example 5

To a mixture of benzhydryl 7-[2-(2-aminothiazol-4-yl)-2-hydroxyiminoacetamido]-3-(1,2,4-thiadiazol-5-yl)-thiomethyl-3-cephem-4-carboxylate (430 g) and anisole (430 ml) in dichloromethane (1.7 ℓ) was added dropwise trifluoroacetic acid (860 ml) under ice-cooling. The mixture was stirred for 2 hours at 5-10°C. The reaction mixture was poured into diisopropyl ether (21.5 ℓ) to be pulverized. The precipitates were collected by filtration, washed with diisopropyl ether and dried over phosphorus pentoxide to give a yellow powder. The powder (400 g) was suspended in water and adjusted to pH 8 with saturated aqueous sodium hydrogen carbonate.

The insoluble materials were filtered off and the filtrate was adjusted to pH 6.0 with 1N-hydrochloric acid. The solution was subjected to a column chromatography on a non-ionic adsorption resin "Diaion HP-20" using 35% aqueous isopropyl alcohol as an eluent. Fractions containing the desired compound were collected and adjusted to pH 2.2 with 10% aqueous hydrochloric acid. The resulting precipitates were collected by filtration, washed with ice-water and dried over phosphorus pentoxide to give 7-[2-(2-aminothiazol-4-yl)-2-hydroxyiminoacetamido]-3-(1,2,4-thiadiazol-5-yl)thiomethyl-3-cephem-4-carboxylic acid (syn isomer) (98.9 g).

IR (Nujol) : 3200, 1750, 1690, 1650 $cm^{-1}$

NMR ($DMSO-d_6$, δ) : 3.52 and 3.78 (2H, ABq, J=18Hz), 4.30 and 4.62 (2H, ABq, J=13Hz), 5.13 (1H, d, J=5Hz), 5.78 (1H, d-d, J=5Hz, 8Hz), 6.65 (1H, s), 7.07 (2H, br s), 8.70 (1H, s), 9.42 (1H, d, J=8Hz), 11.36 (1H, br s)

Example 6

To a solution of 2-(2-aminothiazol-4-yl)-2-acetoxyiminoacetic acid (syn isomer) (0.2 g) in tetrahydrofuran (25 ml) was added anhydrous 1-hydroxy-1H-benzotriazole (0.135 g) and N,N'-dicyclohexylcarbodiimide (0.182 g). After the mixture was stirred for 1 hour, at room temperature the precipitate was filtered off. To the filtrate was added benzhydryl 7-amino-3-(1,2,4-thiadiazol-5-yl)thiomethyl-3-cephem-4-carboxylate (0.43 g) at room temperature. The solvent was distilled off and the residue was pulverized in diisopropyl ether to give benzhydryl 7-[2-(2-aminothiazol-4-yl)-2-acetoxyiminoacetamido]-3-(1,2,4-thiadiazol-5-yl)-thiomethyl-3-cephem-4-carboxylate (syn isomer) (0.7 g).

NMR ($DMSO-d_6$, δ) : 2.18 (3H, s), 3.17-4.67 (4H, m), 5.17-6.17 (2H, m), 7.0 (1H, s), 7.12 (1H, s), 7.22-7.73 (10H, m), 8.57 (1H, s)

Example 7

To a suspension of benzhydryl 7-[2-(2-aminothiazol-4-yl)-2-acetoxyiminoacetamido]-3-(1,2,4-thiadiazol-5-yl)-thiomethyl-3-cephem-4-carboxylate (syn isomer) (0.7 g) in a mixture of dichloromethane (3 ml) and anisole (0.7 ml) was added trifluoroacetic acid (1.4 ml) under ice-cooling. The mixture was stirred for 1 hour at the same temperature. The reaction mixture was poured into diisopropyl ether (70 ml). The precipitates were collected by filtration and washed with diisopropyl ether. The resulting powder was poured into water, adjusted to pH 6 with saturated aqueous sodium hydrogen carbonate and washed with ethyl acetate. The aqueous solution was adjusted to pH 3.5 with 1N-hydrochloric acid and extracted with ethyl acetate. The solvent was distilled off and the residue was pulverized in diisopropyl ether to give 7-[2-(2-aminothiazol-4-yl)-2-acetoxyimino-acetamido]-3-(1,2,4-thiadiazol-5-yl)thiomethyl-3-cephem-4-carboxylic acid (syn isomer) (0.1 g).

IR (Nujol) : 1760-1770, 1660, 1530 $cm^{-1}$

NMR ($DMSO-d_6$, $\delta$) : 2.15 (3H, s), 3.67 (2H, ABq, J=18Hz), 4.28 and 4.59 (2H, ABq, J=13Hz), 5.15 (1H, d, J=5Hz), 5.78 (1H, d-d, J=5Hz, 8Hz), 7.01 (1H, s), 7.10-7.43 (2H, br s), 8.64 (1H, s), 9.76 (1H, d, J=8Hz)

Example 8

A suspension of 7-[2-(2-aminothiazol-4-yl)-2-hydroxyiminoacetamido]-3-acetoxymethyl-3-cephem-4-carboxylic acid (syn isomer) (1 g) in water (30 ml) was adjusted to pH 6.3 with saturated aqueous solution of sodium hydrogen carbonate. To the solution was added potassium 1,2,4-thiadiazol-5-thiolate at room temperature with keeping a range of pH 6.0 - pH 6.5. The mixture was stirred for 5 hours at 60 - 65°C.

The precipitates in the reaction mixture were filtered off and the remaining aqueous solution was adjusted to pH 3 with 1N-hydrochloric acid. The precipitates were collected by filtration and dried over phosphorus pentoxide to give 7-[2-(2-aminothiazol-4-yl)-2-hydroxyiminoacetamido]-3-(1,2,4-thiadiazol-5-yl)thiomethyl-3-cephem-4-carboxylic acid (syn isomer) (0.37 g).

IR (Nujol) : 3200, 1750, 1690, 1650 $cm^{-1}$

NMR (DMSO-$d_6$, δ) : 3.52 and 3.78 (2H, ABq, J=18Hz), 4.30 and 4.62 (2H, ABq, J=13Hz), 5.13 (1H, d, J=5Hz), 5.78 (1H, d-d, J=5Hz, 8Hz), 6.65 (1H, s), 7.07 (2H, br s), 8.70 (1H, s), 9.42 (1H, d, J=8Hz), 11.36 (1H, br s)

Example 9

To a solution of 7-[2-(2-aminothiazol-4-yl)-2-hydroxyiminoacetamido]-3-(1,2,4-thiadiazol-5-yl)thiomethyl-3-cephem-4-carboxylic acid (1.0 g) in N,N-dimethylformamide (20 ml) was added cesium carbonate (0.39 g) at room temperature. The mixture was stirred at the same temperature under reduced pressure for 20 minutes and to the mixture was added pivaloyloxymethyl iodide (0.27 g) under ice-cooling. After stirring at the same temperature for 15 minutes, the reaction mixture was poured into a mixture of ethyl acetate (200 ml) and water (100 ml). The separated organic layer was washed with water, 5% an aqueous solution of sodium bicarbonate and brine, and dried over magnesium sulfate. The solution was evaporated and the residue was pulverized with isopropyl ether to give pivaloyloxymethyl 7-[2-(2-aminothiazol-4-yl)-2-hydroxyiminoacetamido]-3-(1,2,4-thiadiazol-5-yl)thiomethyl-3-cephem-4-carboxylate (syn isomer) (0.57 g).

IR (Nujol) : 3250, 1780, 1740, 1680 $cm^{-1}$

NMR (DMSO-$d_6$, δ) : 1.15 (9H, s), 3.54 and 3.83

(2H, ABq, J=18Hz), 4.20, 4.60 (2H, ABq, J=13Hz), 5.15 (1H, d, J=5Hz), 5.84 (1H, d-d, J=5, 8Hz), 5.90 (2H, ABq, J=6Hz), 6.62 (1H, s), 8.66 (1H, s), 9.35 (1H, d, J=8Hz)

Example 10

The following compounds were obtained according to a similar manner to that of Example 2.

(1) 7-[2-(2-Aminothiazol-4-yl)-2-hydroxyiminoacetamido]-3-(1,2,4-thiadiazol-5-yl)thiomethyl-3-cephem-4-carboxylic acid (syn isomer).

IR (Nujol) : 3200, 1750, 1690, 1650 $cm^{-1}$

NMR (DMSO-$d_6$, δ) : 3.52 and 3.78 (2H, ABq, J=18Hz), 4.30 and 4.62 (2H, ABq, J=13Hz), 5.13 (1H, d, J=5Hz), 5.78 (1H, d-d, J=5Hz, 8Hz), 6.65 (1H, s), 7.07 (2H, br s), 8.70 (1H, s), 9.42 (1H, d, J=8Hz), 11.36 (1H, br s)

(2) Benzhydryl 7-[2-(2-aminothiazol-4-yl)-2-hydroxyiminoacetamido]-3-(1,2,4-thiadiazol-5-yl)-thiomethyl-3-cephem-4-carboxylate (syn isomer).

IR (Nujol) : 1780, 1720, 1665, 1620 $cm^{-1}$

NMR (DMSO-$d_6$, δ) : 3.59 and 3.83 (2H, ABq, J=18Hz), 4.23 and 4.58 (2H, ABq, J=13Hz), 5.23 (1H, d, J=5Hz), 5.90 (1H, d-d, J=5Hz, 8Hz), 6.67 (1H, s), 7.00-7.80 (10H, m), 8.55 (1H, s), 9.46 (1H, d, J=8Hz), 11.40 (1H, s)

(3) 7-[2-(2-Aminothiazol-4-yl)-2-acetoxyiminoacetamido]-3-(1,2,4-thiadiazol-5-yl)thiomethyl-3-cephem-4-carboxylic acid (syn isomer).

IR (Nujol) : 1760-1770, 1660, 1530 $cm^{-1}$

NMR (DMSO-$d_6$, δ) : 2.15 (3H, s), 3.67 (2H, ABq, (J=18Hz), 4.28 and 4.59 (2H, ABq, J=13Hz),

5.15 (1H, d, J=5Hz), 5.78 (1H, d-d, J=5Hz, 8Hz), 7.01 (1H, s), 7.10-7.43 (2H, br s), 8.64 (1H, s), 9.76 (1H, d, J=8Hz)

(4) Pivaloyloxymethyl 7-[2-(2-aminothiazol-4-yl)-2-hydroxyiminoacetamido]-3-(1,2,4-thiadiazol-5-yl)-thiomethyl-3-cephem-4-carboxylate (syn isomer).

IR (Nujol) : 3250, 1780, 1740, 1680 $cm^{-1}$

NMR ($DMSO-d_6$, δ) : 1.15 (9H, s), 3.54 and 3.83 (2H, ABq, J=18Hz), 4.20, 4.60 (2H, ABq, J=13Hz), 5.15 (1H, d, J=5Hz), 5.84 (1H, d-d, J=5, 8Hz), 5.90 (2H, ABq, J=6Hz), 6.62 (1H, s), 8.66 (1H, s), 9.35 (1H, d, J=8Hz)

Example 11

The following compound was obtained according to a similar manner to that of Example 1.

Pivaloyloxymethyl 7-[2-(2-aminothiazol-4-yl)-2-hydroxyiminoacetamido]-3-(1,2,4-thiadiazol-5-yl)-thiomethyl-3-cephem-4-carboxylate (syn isomer).

IR (Nujol) : 3250, 1780, 1740, 1680 $cm^{-1}$

NMR ($DMSO-d_6$, δ) : 1.15 (9H, s), 3.54 and 3.83 (2H, ABq, J=18Hz), 4.20, 4.60 (2H, ABq, J=13Hz), 5.15 (1H, d, J=5Hz), 5.84 (1H, d-d, J=5, 8Hz), 5.90 (2H, ABq, J=6Hz), 6.62 (1H, s), 8.66 (1H, s), 9.35 (1H, d, J=8Hz)

Example 12

The following compounds were obtained according to a similar manner to that of Example 3.

7-[2-(2-Aminothiazol-4-yl)-2-acetoxyiminoacetamido]-3-(1,2,4-thiadiazol-5-yl)thiomethyl-3-cephem-4-carboxylic acid (syn isomer).

IR (Nujol) : 1760-1770, 1660, 1530 $cm^{-1}$
NMR ($DMSO-d_6$, δ) : 2.15 (3H, s), 3.67 (2H, ABq, J=18Hz), 4.28 and 4.59 (2H, ABq, J=13Hz), 5.15 (1H, d, J=5Hz), 5.78 (1H, d-d, J=5Hz, 8Hz), 7.01 (1H, s), 7.10-7.43 (2H, br s), 8.64 (1H, s), 9.76 (1H, d, J=8Hz)

Example 13

The following compounds were obtained according to a similar manner to that of Example 8.

(1) Benzhydryl 7-[2-(2-tetrahydropyranyloxyimino)-2-(2-tritylaminothiazol-4-yl)acetamido]-3-(1,2,4-thiadiazol-5-yl)thiomethyl-3-cephem-4-carboxylate (syn isomer).

IR (Nujol) : 3350, 1770, 1700, 1660 $cm^{-1}$
NMR ($DMSO-d_6$, δ) : 1.50-2.00 (m), 3.50-4.30 (4H, m), 5.30 (1H, d, J=5Hz), 5.85 (1H, dd, J=5Hz, J=8Hz), 6.83 (1H, s), 7.03 (1H, s), 7.10-7.77 (m), 8.57 (1H, s), 9.67 (1H, d, J=8Hz)

(2) Benzhydryl 7-[2-(2-aminothiazol-4-yl)-2-hydroxyiminoacetamido]-3-(1,2,4-thiadiazol-5-yl)thiomethyl-3-cephem-4-carboxylate (syn isomer).

IR (Nujol) : 1780, 1720, 1665, 1620 $cm^{-1}$
NMR ($DMSO-d_6$, δ) : 3.59 and 3.83 (2H, ABq, J=18Hz), 4.23 and 4.58 (2H, ABq, J=13Hz), 5.23 (1H, d, J=5Hz), 5.90 (1H, d-d, J=5Hz, 8Hz), 6.67 (1H, s), 7.00-7.80 (10H, m), 8.55 (1H, s), 9.46 (1H, d, J=8Hz), 11.40 (1H, s)

(3) Benzhydryl 7-[2-(2-aminothiazol-4-yl)-2-acetoxyiminoacetamido]-3-(1,2,4-thiadiazol-5-yl)-thiomethyl-3-cephem-4-carboxylate (syn isomer).

NMR (DMSO-$d_6$, δ) : 2.18 (3H, s), 3.17-4.67 (4H, m), 5.17-6.17 (2H, m), 7.0 (1H, s), 7.12 (1H, s), 7.22-7.73 (10H, m), 8.57 (1H, s)

(4) 7-[2-(2-Aminothiazol-4-yl)-2-acetoxyimino-acetamido]-3-(1,2,4-thiadiazol-5-yl)thiomethyl-3-cephem-4-carboxylic acid (syn isomer).

IR (Nujol) : 1760-1770, 1660, 1530 $cm^{-1}$

NMR (DMSO-$d_6$, δ) : 2.15 (3H, s), 3.67 (2H, ABq, J=18Hz), 4.28 and 4.59 (2H, ABq, J=13Hz), 5.15 (1H, d, J=5Hz), 5.78 (1H, d-d, J=5Hz, 8Hz), 7.01 (1H, s), 7.10-7.43 (2H, br s), 8.64 (1H, s), 9.76 (1H, d, J=8Hz)

(5) Pivaloyloxymethyl 7-[2-(2-aminothiazol-4-yl)-2-hydroxyiminoacetamido]-3-(1,2,4-thiadiazol-5-yl)-thiomethyl-3-cephem-4-carboxylate (syn isomer).

IR (Nujol) : 3250, 1780, 1740, 1680 $cm^{-1}$

NMR (DMSO-$d_6$, δ) : 1.15 (9H, s), 3.54 and 3.83 (2H, ABq, J=18Hz), 4.20, 4.60 (2H, ABq, J=13Hz), 5.15 (1H,d, J=5Hz), 5.84 (1H, d-d, J=5, 8Hz), 5.90 (2H, ABq, J=6Hz), 6.62 (1H, s), 8.66 (1H, s), 9.35 (1H, d, J=8Hz)

(to be continued to the next page)

0214462

Example 14

To a suspension of 7-[2-(2-aminothiazol-4-yl)-2-hydroxyiminoacetamido]-3-(1,2,4-thiadiazol-5-yl)thiomethyl-3-cephem-4-carboxylic acid (syn isomer) (5.0 g) in water (50 ml) was added sodium bicarbonate (924 mg) under stirring. The resulting solution was subjected to column chromatography on aluminum oxide (50 g) (Woelm Pharma Co., type W200) using water as an eluant. The eluate was lyophilized to give sodium 7-[2-(2-aminothiazol-4-yl)-2-hydroxyiminoacetamido]-3-(1,2,4-thiadiazol-5-yl)thiomethyl-3-cephem-4-carboxylate (syn isomer) (4.2 g).

IR (Nujol): 3300, 1750, 1650, 1600, 1520 $cm^{-1}$

Example 15

To a suspension of 7-[2-(2-aminothiazol-4-yl)-2-hydroxyiminoacetamido]-3-(1,2,4-thiadiazol-5-yl)thiomethyl-3-cephem-4-carboxylic acid (syn isomer) (5.0 g) in methanol (25 ml) was added 5 molar sulfuric acid (2 ml) with stirring at room temperature. The resulting solution was poured into diethyl ether (600 ml). The precipitates were collected by filtration and washed with diethyl ether to give 7-[2-(2-aminothiazol-4-yl)-2-hydroxyiminoacetamido]-3-(1,2,4-thiadiazol-5-yl)thiomethyl-3-cephem-4-carboxylic acid sulfate (syn isomer) (5.6 g).

IR (Nujol): 1770, 1640, 1590, 1520 $cm^{-1}$
NMR (DMSO-$d_6$, $\delta$): 3.60 and 3.80 (2H, ABq, J=18Hz), 4.40 and 4.60 (2H, ABq, J=14Hz), 5.20 (1H, d, J=5Hz), 5.80 (1H, dd, J=5Hz, 8Hz), 6.87 (1H, s), 8.70 (1H, s), 9.68 (1H, d, J=8Hz)

Example 16

(1) To a suspension of 7-[2-(2-aminothiazol-4-yl)-2-hydroxyiminoacetamido]-3-(1,2,4-thiadiazol-5-yl)thiomethyl-3-cephem-4-carboxylic acid (syn isomer) (10.0 g) in methanol (40 ml) was added conc. hydrochloric acid (2.0 ml) with stirring at room temperature. The resulting solution was poured into diethyl ether (500 ml). The precipitates were collected by filtration and washed with diethyl ether to give 7-[2-(2-aminothiazol-4-yl)-2-hydroxyiminoacetamido]-3-(1,2,4-thiadiazol-5-yl)thiomethyl-3-cephem-4-carboxylic acid hydrochloride (syn isomer) (10.6 g).

mp: 160 - 165°C (dec.)
IR (Nujol): 1780, 1680, 1635, 1530 $cm^{-1}$
NMR ($DMSO-d_6$, δ): 3.70 (2H, m), 4.34 and 4.71 (2H, ABq, J=13Hz), 5.21 (1H, d, J=5Hz), 5.80 (1H, dd, J=5Hz, 8Hz), 6.87 (1H, s), 8.72 (1H, s), 9.71 (1H, d, J=8Hz)

(2) The suspension of 7-[2-(2-aminothiazol-4-yl)-2-hydroxyiminoacetamido]-3-(1,2,4-thiadiazol-5-yl)thiomethyl-3-cephem-4-carboxylic acid hydrochloride (syn isomer) (400 mg) in acetonitrile (4 ml) was heated at 45°C for 2 hours with stirring. After cooling, the precipitates were collected by filtration and washed with acetonitrile and then diethyl ether to give the crystals of 7-[2-(2-aminothiazol-4-yl)-2-hydroxyiminoacetamido]-3-(1,2,4-thiadiazol-5-yl)thiomethyl-3-cephem-4-carboxylic acid hydrochloride (syn isomer) (410 mg). The crystals contain one molar equivalent acetonitrile.

mp: 80 - 84°C (dec.)
IR (Nujol): 3260 - 3050, 2240, 1770, 1710, 1650, 1630, 1540 $cm^{-1}$

NMR ($DMSO-d_6$, δ): 2.05 (3H, s), 3.66 (2H, m), 4.31 and 4.81 (2H, ABq, J=13Hz), 5.14 (1H, d, J=5Hz), 5.73 (1H, dd, J=5Hz, 8Hz), 6.80 (1H, s), 8.64 (1H, s), 9.60 (1H, d, J=8Hz)

0214462

What we claim is :

1. A cephem compound of the formula :

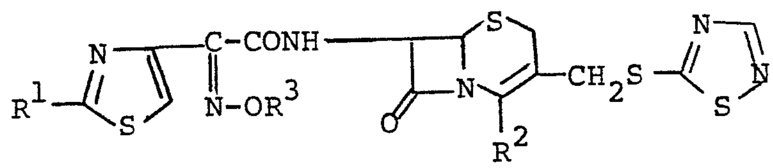

wherein $R^1$ is amino or protected amino,
$R^2$ is carboxy or protected carboxy, and
$R^3$ is hydrogen or hydroxy-protective group,
and pharmaceutically acceptable salts thereof.

2. A compound of claim 1, wherein
$R^1$ is amino or ar(lower)alkylamino,
$R^2$ is carboxy or esterified carboxy and
$R^3$ is hydrogen, acyl or tetrahydropyranyl.

3. A compound of claim 2, wherein
$R^1$ is amino and $R^3$ is hydrogen.

4. A compound of claim 3, which is
7-[2-(2-aminothiazol-4-yl)-2-hydroxyiminoacetamido]-3-(1,2,4-thiadiazol-5-yl)thiomethyl-3-cephem-4-carboxylic acid (syn isomer) or its sodium salt.

5. A compound of claim 3, wherein
$R^2$ is esterified carboxy.

6. A compound of claim 5, wherein
$R^2$ is ar(lower)alkoxycarbonyl or lower alkanoyloxy-(lower)alkoxycarbonyl.

7. A compound of claim 6, which is
benzhydryl 7-[2-(2-aminothiazol-4-yl)-2-hydroxyiminoacetamido]-3-(1,2,4-thiadiazol-5-yl)-thiomethyl-3-cephem-4-carboxylate (syn isomer).

8. A compound of claim 6, which is pivaloyloxymethyl 7-[2-(2-aminothiazol-4-yl)-2-hydroxyiminoacetamido]-3-(1,2,4-thiadiazol-5-yl)-thiomethyl-3-cephem-4-carboxylate (syn isomer).

9. A compound of claim 2, wherein $R^3$ is lower alkanoyl or tetrahydropyranyl.

10. A compound of claim 9, which is selected from 7-[2-(2-aminothiazol-4-yl)-2-acetoxyiminoacetamido]-3-(1,2,4-thiadiazol-5-yl)thiomethyl-3-cephem-4-carboxylic acid (syn isomer), benzhydryl 7-[2-(2-aminothiazol-4-yl)-2-acetoxyiminoacetamido]-3-(1,2,4-thiadiazol-5-yl)-thiomethyl-3-cephem-4-carboxylate (syn isomer) and benzhydryl 7-[2-(2-tetrahydropyranyloxyimino)-2-(2-tritylaminothiazol-4-yl)acetamido]-3-(1,2,4-thiadiazol-5-yl)thiomethyl-3-cephem-4-carboxylate (syn isomer).

11. A process for production of a cephem compound of the formula :

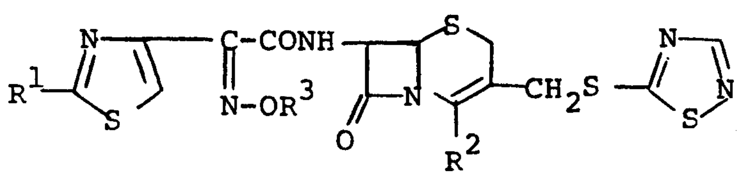

wherein $R^1$ is amino or protected amino,
$R^2$ is carboxy or protected carboxy, and
$R^3$ is hydrogen or hydroxy-protective group,
or a salt thereof,
which comprises

i) reacting a compound of the formula :

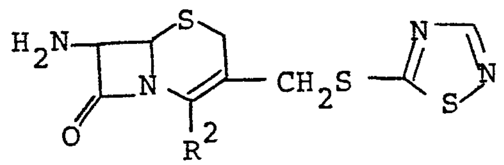

wherein $R^2$ is as defined above, or its reactive derivative at the amino group or a salt thereof, with a compound of the formula :

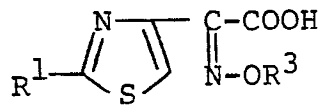

wherein $R^1$ and $R^3$ are each as defined above, or its reactive derivative at the carboxy group or a salt thereof, to give a compound of the formula:

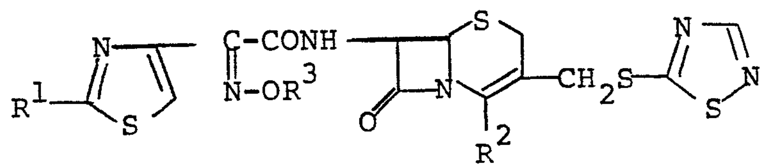

wherein $R^1$, $R^2$ and $R^3$ are each as defined above, or a salt thereof;

ii) subjecting a compound of the formula:

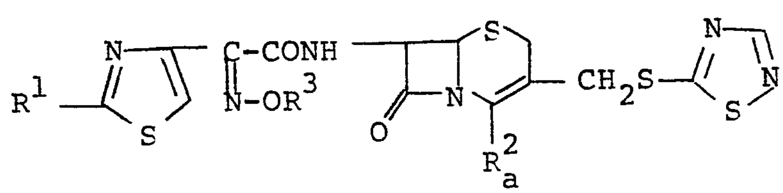

wherein $R^1$ and $R^3$ are each as defined above,

and

$R^2_a$ is protected carboxy,

or a salt thereof, to elimination reaction of the carboxy-protective group, to give a compound of the formula :

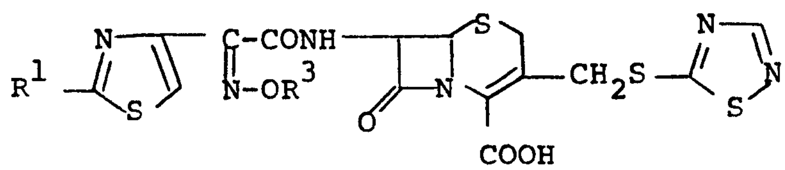

wherein $R^1$ and $R^3$ are each as defined above, or a salt thereof;

iii) reacting a compound of the formula:

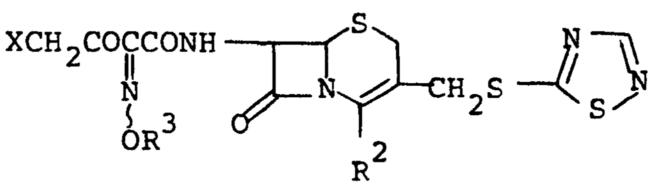

wherein $R^2$ and $R^3$ are each as defined above,

and

X is an acid residue,

or a salt thereof, with a compound of the formula:

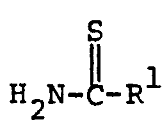

wherein $R^1$ is as defined above,
to give a compound of the formula :

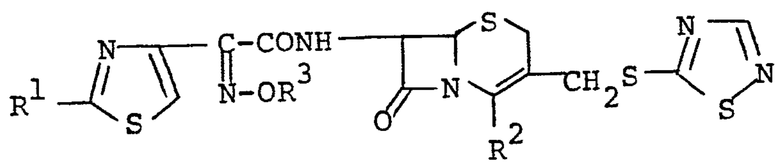

wherein $R^1$, $R^2$ and $R^3$ are each as defined above, or a salt thereof;

iv) subjecting a compound of the formula :

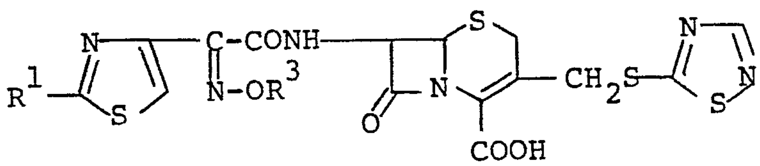

wherein $R^1$ and $R^3$ are each as defined above, or a salt thereof, to esterification reaction, to give a compound of the formula:

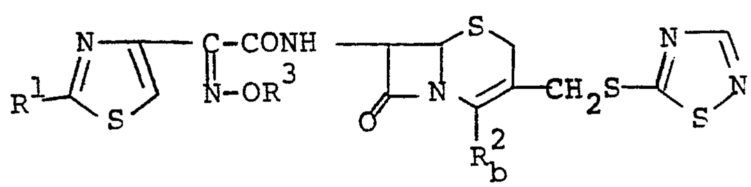

wherein $R^1$ and $R^3$ are each as defined above, and $R_b^2$ is esterified carboxy or a salt thereof;

v) subjecting a compound of the formula :

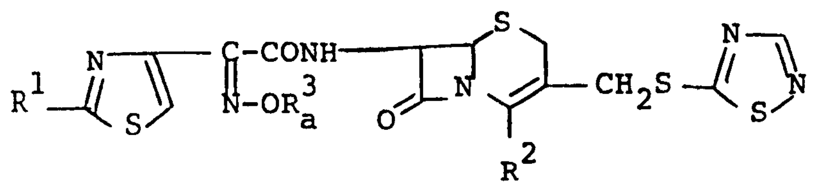

wherein $R^1$ and $R^2$ are each as defined above,

and

$R_a^3$ is hydroxy-protective group,

or a salt thereof, to elimination reaction of the hydroxy-protective group, to give a compound of the formula:

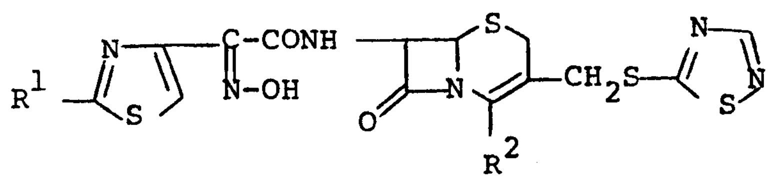

wherein $R^1$ and $R^2$ are each as defined above, or a salt thereof;

vi) subjecting a compound of the formula:

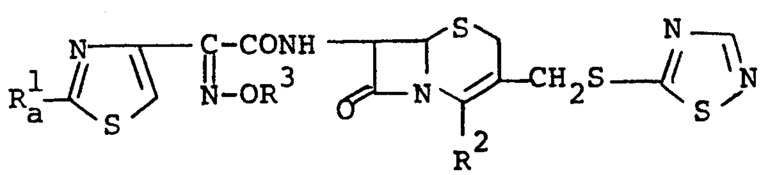

wherein $R^2$ and $R^3$ are each as defined above,

and

$R_a^1$ is protected amino,

or a salt thereof, to elimination reaction of the amino-protective group, to give a compound of the formula:

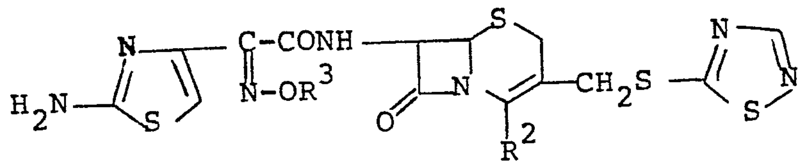

wherein $R^2$ and $R^3$ are each as defined above, or a salt thereof; and

vii) reacting a compound of the formula:

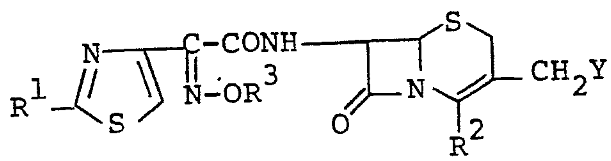

wherein $R^1$, $R^2$ and $R^3$ are each as defined above, and

Y is a group which is replaceable with the formula: 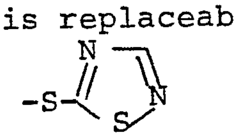

or a salt thereof, with a compound of the formula:

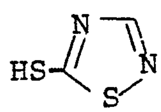

or its reactive derivative at the mercapto group, to give a compound of the formula:

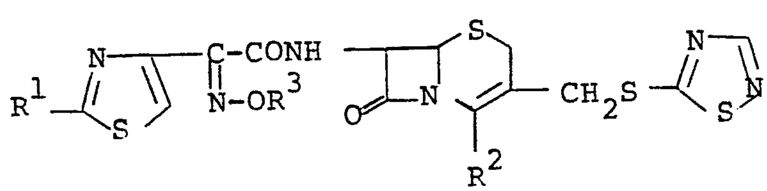

wherein $R^1$, $R^2$ and $R^3$ are each as defined above, or a salt thereof.

11. A pharmaceutical composition which comprises, as an active ingredient, a compound of claim 1 and a pharmaceutically acceptable salt thereof in admixture with pharmaceutically acceptable carriers.

12. Use of a compound of claim 1 as a medicament.

13. Use of a compound of claim 1 and pharmaceutically acceptable salt thereof for treatment of microbial infections.

14. A compound of the formula:

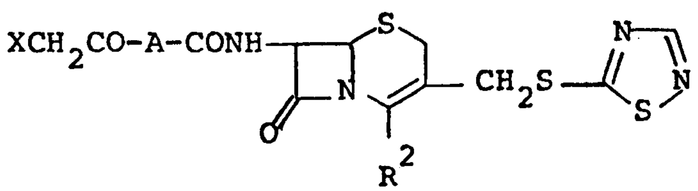

wherein $R^2$ is carboxy or protected carboxy,
A is methylene or a group of the formula: $=N\sim OR^3$, in which $R^3$ is hydrogen or hydroxy-protective group, and
X is an acid residue,
or a salt thereof.

15. A process for the preparation of a compound of the formula:

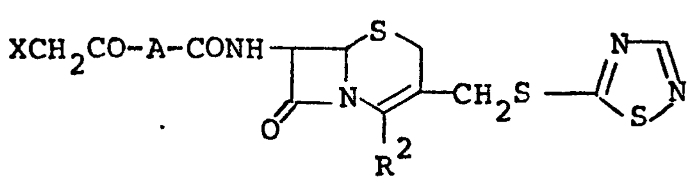

wherein $R^2$ is carboxy or protected carboxy,

A is methylene or a group of the formula: $=N\sim OR^3$, in which $R^3$ is hydrogen or hydroxy-protective group, and

X is an acid residue,

or a salt thereof, which comprises

i) reacting a compound of the formula:

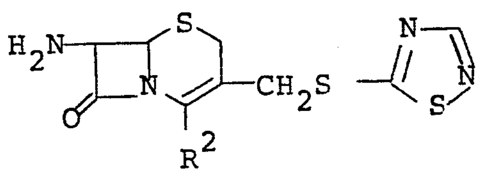

wherein $R^2$ is as defined above, or its reactive derivative at the amino group or a salt thereof, with a compound of the formula:

$XCH_2CO-A-COOH$

wherein A and X are each as defined above, or its reactive derivative at the carboxy group, or a salt thereof, to give a compound of the formula:

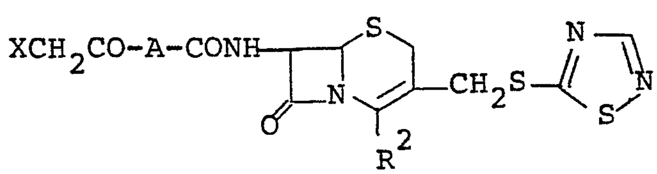

wherein $R^2$, A and X are each as defined above, or a salt thereof; and

ii) reacting a compound of the formula:

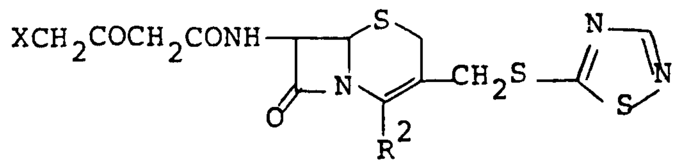

wherein $R^2$ and X are each as defined above, or a salt thereof, with a nitrosating agent to give a compound of the formula:

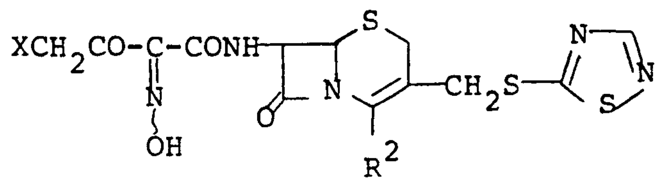

wherein $R^2$ and X are each as defined above, or a salt thereof.

Dr. rer. nat. D. TÜRK · Dipl.-Ing. Ch. GILLE · Dr. rer. nat. U. HRABAL

PATENTANWÄLTE · EUROPEAN PATENT ATTORNEYS

0214462

European Patent Office
Erhardtstr. 27

8000 München 2

EPA EPO-OEB
DG 1
Reçu
21 OKT. 1986

Telefon (0211) 715039
Telefax (0211) 719766
Telex 8582841 rrtg d
Telegramme: Returgi Düsseldorf

Bruckner Straße 20
D-4000 Düsseldorf 13

Ihr Zeichen
Your Ref

Unser Zeichen
Our Ref
T 55 536

Datum
Date
October 9, 1986 - cm

European Patent Application 86 110 728.2
Fujisawa Pharmaceutical Co., ltd.

In reply to the Communciation of October 1, 1986 we apologize for the error and enclose herewith copies of pages 8 to 10 of the claims, in which we renumbered claims 11 to 15 to new claims 12 to 16.

The additional claim fee of 65 DM is paid today.

ZUR KASSE

Türk Gille Hrabal
Patentanwälte

durch:

Patentanwalt

Encl.